# EUROPEAN PATENT APPLICATION

(11) **EP 4 477 197 A1**
(43) Date of publication of application: **18.12.2024**
(21) Application number: 23179119.5
(22) Date of filing: 14.06.2023
(51) Int. Cl.: A61F 11/08, A61F 11/12

(54) **AN EAR INSERT RETAINER**

(71) Applicant: Loop, 2600 Berchem (BE)
(72) Inventor: LANSSIERS, Alyssa Lesley K, 3020 Herent (BE); HAAZEN, Louise Nathalie G, 2018 Antwerpen (BE); VAN VELTHOVEN, Robrecht, 2140 Borgerhout (BE); GRAINGER, William, 2600 Berchem (BE); DE BONDT, Stijn, 2800 Mechelen (BE); VANCOPPENOLLE, Ward Antoon J, 2800 Mechelen (BE); THUYSBAERT, Orlando Michael M, 2018 Antwerpen (BE)
(74) Representative: Hoyng Rokh Monegier B.V.

(57) **Abstract**

An ear insert retainer (1) is provided. The retainer comprises a flexible elongate object (10) for linking two ear inserts, which flexible elongate object has a first and a second end point. Each of the first end point are adapted to be connected to a rearward part of an ear insert (300). The ear insert retainer comprises at least a first interrupting means (100) along the flexible elongate object, this first interrupting means being adapted to interrupt the link of the ear insert with the flexible elongate object when a force applies to the flexible elongate object in the longitudinal direction.

## Description

### Field of the Invention

The present invention generally relates to ear insert retainers, and methods to wear ear inserts, like ear plugs.

### Background of the Invention

Sound and noise can no longer be ignored in today's world. There is a great need for hearing protection against background noise and against excessive noise nuisance. That is why ear inserts are very often used in all kinds of circumstances such as in the office, in noisy factory halls, on public transport, even at music festivals or in the bedroom. The danger with these ear inserts is that the user may lose an ear insert, and thereby is involuntarily exposed to noise or sound.

There are sets of ear inserts for which the ear inserts are connected to each other with a cord or retainer. These cords are each often moulded into the material from which the ear insert is made and are an integral part of the ear insert itself. However, the noise generated by the rubbing of the cord on the clothing of the user himself can be a source of disturbing noise. In addition, forces accidentally applied to the lanyard could rip the ear inserts out of the ears of the user and may cause damage to the user.

### Summary of the Invention

According to a first aspect of the invention, an ear insert retainer is provided. The retainer comprises a flexible elongate object for linking two ear inserts, which flexible elongate object has a first and a second end point. Each of the first end point are adapted to be connected to a rearward part of an ear insert. The ear insert retainer comprises at least a first interrupting means along the flexible elongate object, this first interrupting means being adapted to interrupt the link of the ear insert with the flexible elongate object when a force applies to the flexible elongate object in the longitudinal direction.

The force applied to the flexible elongate object in the longitudinal direction to cause said interruption, preferably is more than or equal to 1 Newton, more preferably to more than or equal to 2 Newton or even more than or equal to 3 Newton. The force applied to the flexible elongate object in the longitudinal direction to cause said interruption, preferably does not exceed 5 Newton, most preferably does not exceed 3 Newton. Hence the force, hereinafter the interruption force, necessary to interrupt the link of the ear inserts when said force applies to the flexible elongate object in the longitudinal direction, is in the range of 1 Newton to 5 Newton, and preferably in the range of 2 Newton to 3 newton.

Preferably, each of the first end point is adapted to be movably and detachably connected to a rearward part of an ear insert.

The ear inserts are preferably ear inserts for attenuation of ambient noise or for protection ears from excessive noise exposure.

The flexible elongate object may have the interrupting means along the length of the cord. The interruption means may comprise a first and a second aglet, each being coupled to an end of the flexible elongate object at the position where the interrupting means is present. The first aglet and the second aglet each may comprise one of a first and a second half of a pair of mating connectors, which pair is chosen such that the force to rupture and break the connection is on the one hand low enough to avoid damage when a too large axial force is applied to the flexible elongate object, which too large force may be transferred to the ear inserts connected to the flexible elongate object. The force to rupture and break the connection is on the other hand high enough to avoid disconnection of the two mating connectors when two linked ear inserts are worn in normal circumstances in the ears by a user. The friction force, the shape and the material of the mating connectors may be chosen to this extent in order to tune the force to rupture.

This first half may be a part of a pair of male and female mating connectors. Preferably the first half is the male half of such male and female pair of mating connectors.

The first half may have an axial cross section of a cylinder, preferably having a tapered top. The receiving second half may be a cylindrical recess having an identical or slightly smaller diameter. The connection force will mainly be defined by friction forces between the two halves, and is influenced by amongst others the length of the cylinder, the diameter and the material the two parts are made of. The cylindrical shape may comprise a radially inwards recess in longitudinal direction of the cylinder along the outer surface of the cylinder. The provision of such recess also influences the friction force, but facilitates an easily connection between the two halves.

The first half may have an axial cross section of a mushroom, the cap of the mushroom pointing away from the flexible elongate object. The receiving second half may be provided with a corresponding recess, optionally having a protrusion to snap at the lower part or under the cap of the mushroom. The mushroom shape may comprise a radially inwards recess in longitudinal direction of the mushroom shape along the outer surface of the mushroom shape, in order to facilitate again the plugging in and out. Hence the male half has a mushroom shape, the oblong side of the mushroom being oriented outwards, in the direction of the female half to couple with during connection.

The end points of the flexible elongate object are preferably movably and detachably connected to the rearward end of an ear insert. Optionally by means of a stem, where the flexible elongate object is firmly connected (by e.g. an aglet) to the stem itself.

The flexible elongate object may be a necklace, a wire, a filament, a textile cord or rope, a polymer cord or rope, a chain, a tape, or any other suitable elongate object. The flexible elongate object may be provided from metal, polymer, like polyamide, e.g. PA6, PA69.6 or PA6.12, rubber, silicone, metal, metal chains, and alike. Most preferably the flexible elongate object is a nylon cord or a necklace or chain from metal or metallised polymeric links.

The length of the flexible elongate object may be tuned to the kind of user the retainer is intended for, although in general, the length of the flexible elongate object may be long enough to have the flexible elongate object loosely hanging around the neck of a human being. Such length may preferably be in the range of 35cm to 85cm, more preferred in the range of 45cm to 75cm, like 60cm, 61 cm, 62cm, 64cm, 65cm, 66cm or 67cm. The length may be adaptable by an appropriate adapter which can shorten or lengthen the flexible elongate object, such a sa cord locker or cord stopper, e.g. a double cord locker.

When reference if made to the longitudinal direction of the flexible elongate object, reference is made to the direction in which the object is elongate.

The elongate joining member preferably is a stemlike member, having two outer ends between a relatively slender body. The elongate joining member preferably is provided from relatively flexible material, such as but not limited to polymer, like polyamide, e.g. PA6, PA69.6 or PA6.12, rubber, silicone, NBR (also known as nitril rubber, Buna-N or acrylonitrile butadiene rubber), TPE (i.e. thermoplastic elastomers), TPU (i.e. thermoplastic polyurethane) and high tensile silicone and alike.

When reference is made to an ear insert, reference is made to a device which is suitable to be plugged into or in front of the ear channel. An ear insert has a rearward part and an ear tip. The ear tip is the forward part of ear insert, being adapted to be inserted in or in front of the ear channel. The rearward part is the part extending outwards the ear canal, and fit to be held by a hand while inserting or withdrawing the ear insert, including its ear tip, in and out the ear canal. The

The ear insert retainer of the invention may be used to hold ear inserts at its rearward part. The ear insert may be adapted for filtering, attenuating, and/or partially or completely blocking sound and noise entering the ear channel, i.e. an earplug, but may also be used to bring sound to the ear channel in a controlled way, e.g. being an earbud, an ear headphone, true Wireless in-ear device (also referred to as TWS), Bluetooth ear insert, airpod and alike. The ear insert retainer may be used to retain two, or more, ear inserts, like ear plugs, two earbuds, in ear headphones, true Wireless in-ears (also referred to as TWS), Bluetooth ear inserts, airpods and alike

According to some embodiments, the first interrupting means may be present at the first end point of the flexible elongate object.

According to some embodiments, the first interrupting means may comprise a first member, said first member being adapted to movably and detachably connected the first end point of the flexible elongate object to a rearward part of an ear insert. According to some embodiments, the first member may be a first elongate member having two extremities, the first of its two extremities being adapted to movably and detachably connected said first end point of the flexible elongate object to a rearward part of an ear insert. The first member may be provided from flexible polymeric material, preferably silicone.

According to some embodiments, the first member may be a stem-shaped member.

The stem-shaped member comprises a slender body part between its two extremities. The use of such stem-shaped member, in particularly when provided from polymer, like silicone, has the advantage that the connected ear insert and the flexible elongate object are auditorily disconnected. Noise which may be transferred along the flexible elongate object, e.g. by contact of the flexible elongate object with the body or clothing of the user, may be transferred via the first interrupting means towards the ear insert and hence the sound received by the ear to a lesser extent, possibly even not transferred at all. The noise is attenuated and may even be blocked by the stem-shaped first member. The provision of a movably and detachably connection to a rearward part of an ear insert further improved this attenuation of this noise and this transfer.

According to some embodiments, the first interrupting means may further comprise an aglet for connecting said first interrupting means to the flexible elongate object. The aglet may be provided from polymer or metallic, like metal material. Examples are polyamide or polyester material, thermoplastic polymeric material, steel, stainless steel, aluminium, copper, brass or noble metals like silver and gold. Possibly, the aglet may be provided at its side oriented away from the flexible elongate object, with a first half of pair of mating connectors.

The pair of mating connectors are chosen such that the force to rupture and break the connection is on the one hand low enough to avoid damage when a too large axial force is applied to the flexible elongate object, which too large force may be transferred to the ear inserts connected to the flexible elongate object. The force to rupture and break the connection is on the other hand high enough to avoid disconnection of the two mating connectors when two linked ear inserts are worn in normal circumstances in the ears by a user.

The friction force, the shape and the material of the mating connectors may be chosen to this extent.

This first half may be a part of a pair of male and female mating connectors. Preferably the first half is the male half of such male and female pair of mating connectors.

The first half may have an axial cross section of a cylinder, preferably having a tapered top. The receiving second half may be a cylindrical recess having an identical or slightly smaller diameter. The connection force will mainly be defined by friction forces between the two halves, and is influenced by amongst others the length of the cylinder, the diameter and the material the two parts are made of. The cylindrical shape may comprise a radially inwards recess in longitudinal direction of the cylinder along the outer surface of the cylinder. The provision of such recess also influences the friction force, but facilitates an easily connection between the two halves.

The first half may have an axial cross section of a mushroom, the cap of the mushroom pointing away from the flexible elongate object. The receiving second half may be provided with a corresponding recess, optionally having a protrusion to snap at the lower part or under the cap of the mushroom. The mushroom shape may comprise a radially inwards recess in longitudinal direction of the mushroom shape along the outer surface of the mushroom shape, in order to facilitate again the plugging in and out. Hence the male half has a mushroom shape, the oblong side of the mushroom being oriented outwards, in the direction of the female half to couple with during connection.

According to some embodiments, the first half of pair of mating connectors may be the male half of a male and female pair of connectors, said male half having an axial cross section being mushroom shaped, the cap of the mushroom shape pointing away from the flexible elongate object, said mushroom shape comprising a radially inwards recess in longitudinal direction of the mushroom shape along the outer surface of the mushroom shape.

The provision of this radially inwards recess, preferably along the whole length of the shape, has the advantage that there is less to no noise created when the male and female pair of connectors are disconnected. In absence of such recess, this disconnection may cause a sound to be transferred, optionally even a quite loud sound, towards the ear inserts. The provision of such recess avoids this disconnection sound to a large extent, possibly even cause no such sound to be present upon disconnection.

The male half may be provided from polymer or metallic, like metal material. Examples are polyamide or polyester material, thermoplastic polymeric material, steel, stainless steel, aluminium, copper, brass or noble metals like silver and gold.

The male half may be clamped or glued in the tubular volume of the aglet and provide the outer end of the aglet. Possibly the male half may be pressed into the annular opening of the tubular volume of the aglet. This male half may have a radial thickened ring part which clamps in the tubular volume when pressed into said volume.

At the other end of the aglet, a bush, like a metal, e.g. copper bush, a tapered bush or even a tapered metal, like copper bush may be provided to clamp the tubular volume of the aglet to the outer end of the elongate flexible object. Alternatively, in particularly when the elongate flexible object is a metal object and/or a chain like object, the other end of the aglet is provided with a radial perforation or eye, thought which the outer end of the elongate flexible object, e.g. the last chain of the chain-like elongate flexible object, pierces through.

According to some embodiments, the second half of the mating connectors may be provided at the second extremity of the first member.

This second part may be the female part of a pair of male and female mating connectors. Possibly even preferably this female part is provided from silicone.

Possibly, the second half may be provided from a polymer material, preferably flexible silicone polymer, the material of the male half having a higher hardness as compared to the material of the female half.

Preferably a pair of mating connectors is used where the male half is provided from aluminium, the female half being provided from silicone, or vice versa. It was found that this choice provides suitable friction to achieve a proper rupture force necessary to interrupt the linkage between the ear inserts used in combination with this ear insert retainer. The provision of a silicone female half is preferred, in combination with a harder male half, e.g. from aluminium, as said silicone tend to relax over time, which may lead to early disconnection. It was found that the provision of female halves in silicone may postpone or even avoid this problem.

Preferably silicones with a hardness in the range of 30 to 70 shoreA, like 40 to 60 shoreA, e.g. about 50 shoreA are used.

As to the use of silicone in the context of this invention in general, preferably silicone with a lower hardness is used. It was found that higher flexible silicone, hence silicone with a lower hardness, tend to increase the disconnection forces.

Additionally or alternatively, the pair of mating connectors may comprise a mutually attracting pair of elements, one of said mutually attracting pair of elements being a magnet.

According to some embodiments, the ear insert retainer further may comprise at least a second interrupting means along said flexible elongate object, said second interrupting means being adapted to interrupt the link of the ear inserts when a force applies to the flexible elongate object in the longitudinal direction, said second interrupting means being present at the second end point of the flexible elongate object. Possibly the first and second interrupting means may be identical. Optionally, the at least one of the first and second interrupting means may comprise a magnetic element, the other of said first and second interrupting means comprises an element from magnetically attractable material, optionally being a magnet itself.

The mutually attracting pair of elements is adapted to cause attraction between the two interrupting means when the interrupting means are brought one aside the other.

These elements may be provided in the tubular volume of an aglet, between the side of the aglet connected to the flexible elongate object and the first half, e.g. the male half of a pair of mating connectors.

the elongate joining members each may have a first outer end being provided with an opening adapted to encompass the rearward part of an ear insert, said rearward part being a stemlike volume extending outwards and out of the ear canal when the ear insert is inserted in an ear canal.

According to a second aspect of the invention, an ear insert set is provided, the set comprising at least two ear inserts and at least one ear insert retainer according to the first aspect of the invention.

Each of the ear inserts comprise an ear tip firmly and optionally disconnectedly connected to a rearward part. The rearward part extends from the ear tip outwards an ear channel when in use, and is used to insert or pull out the ear tip from the ear channel. The ear insert retainer according to the first aspect of the invention may be connected, preferably movably and detachably, to a rearward part of an ear insert.

In the context of this invention, movably means that two parts may move one relative to the other, without losing the connection which holds these two parts together. Detachably means that the two parts may be separated one from the other, so each can be manipulated without being hindered or prevented other.

According to a third aspect of the invention an ear insert retainer according to the first aspect of the invention is used to hold at least two ear inserts linked to the other, the ear inserts each being either inserted in an ear channel, or not.

The flexible elongate object of the ear insert retainer may hang loose between the at least two ear inserts when the inserts are inserted in the ears from the user, or may rest on the neck or shoulders of the user. The flexible elongate object of the ear insert retainer rest on the neck or shoulders of the user m when the inserts are not inserted in the ears from the user.

The ear insert retainer may also be used in combination with one or more, e.g. two earbuds, in ear headphones, true Wireless in-ears (also referred to as TWS), and alike. The ear insert retainer of the invention may be used to hold ear inserts for filtering, attenuating, and/or partially or completely blocking sound and noise entering the ear channel, i.e. ear plugs, but may also be used in combination with one or more, e.g. two earbuds, in ear headphones, true Wireless in-ears (also referred to as TWS), Bluetooth ear inserts, airpods and alike

### Brief Description of the Drawings

Fig. 1 illustrates schematically an ear insert retainer according to the first aspect of the invention.
Fig. 2 illustrates schematically a cross section of the ear insert retainer of figure 1.
Fig. 3 illustrates schematically an exploded view of the ear insert retainer of figure 1.
Fig. 4a and 4b illustrate schematically the male half of a pair of mating connectors as used in the ear insert retainer of figures 1 to 3.

In the different figures, the same reference sign refers to the same or a similar feature.

### Detailed Description of Embodiment(s)

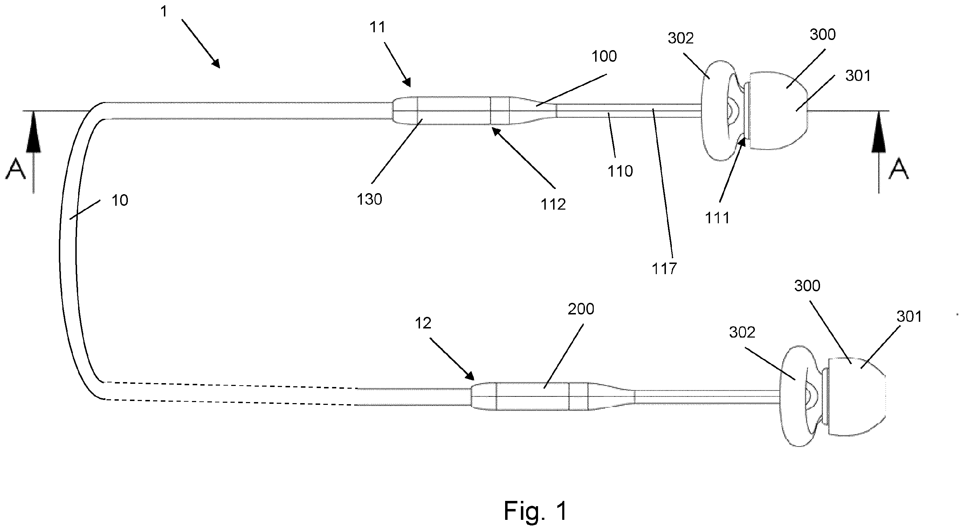

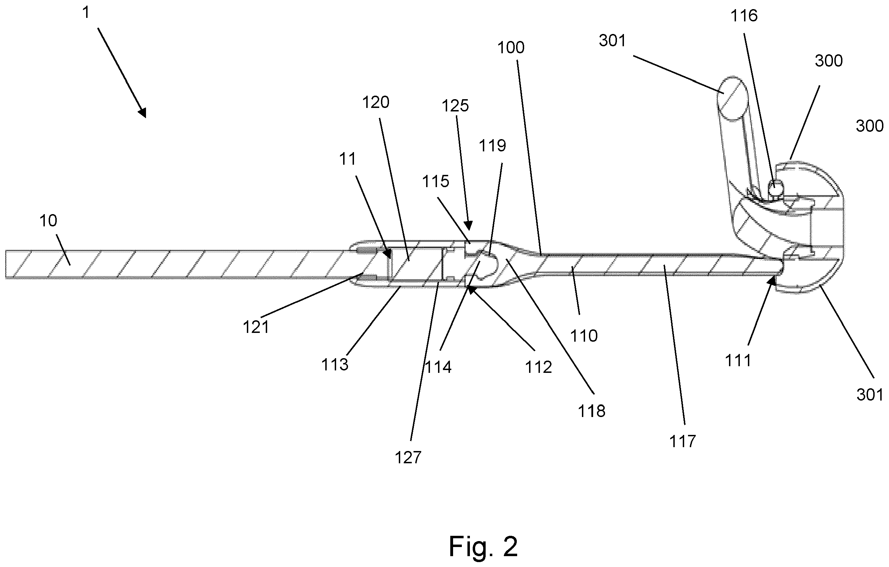

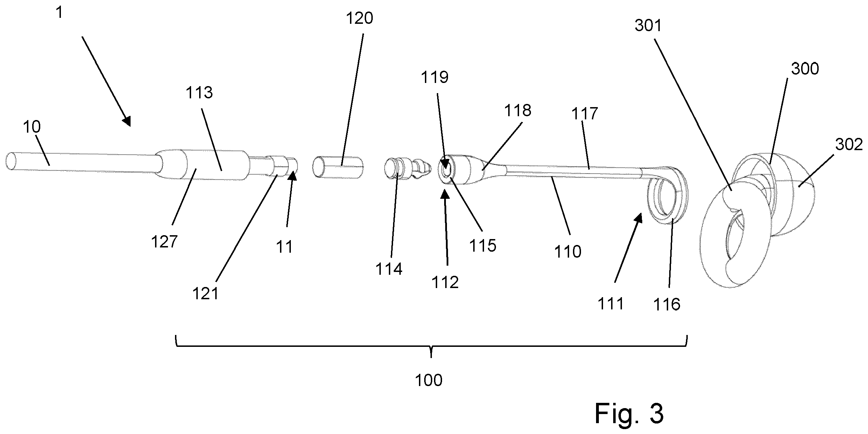

An ear insert retainer 1 according to the first aspect of the invention is shown in figure 1. A cross section of the ear insert retainer according to the plane AA' is shown in figure 2, while an exploded view of this part visible in figure 2, is shown in figure 3.

The ear insert retainer 1 comprises a flexible elongate object, in this embodiment e.g. a polyamide textile cord. The length is about 65cm between the two end points 11 and 12. Each of these two end points are adapted to be connected to a rearward part 301 of an ear insert 300, in this embodiment an ear plug. The first end point is connected to a first ear insert which is to be inserted in an ear channel by its ear tip 302. The rearward part 301 has a stem like part which is inserted in the ear tip, where it may hold the ear tip 301 on this rearward part by the friction forces between the ear tip and the stemlike part of the rearward part 301. In an identical way, the second end point is connected to a second ear insert which is to be inserted in an ear channel as well, in order to wear the ear inserts properly.

To each of the two end points 11 and 12, an interrupting means (100, 200) is provided. Both interrupting means are adapted to interrupt the link of the ear insert with the flexible elongate object when a force applies to the flexible elongate object in the longitudinal direction. The two interrupting means (100, 200) are identical, the first 100 being connected to the first end point 11, the second 200 being connected tot the second end point 12.

Each of the two interrupting means comprise a first member 110 which is movably and detachably connected the end point 11 respectively 12, of the flexible elongate object 10 to a rearward part 301 of an ear insert 300. The movable and detachable connection is provided by providing an elongatable ring at the first extremity 111 of the first member 110, which may be pulled over and placed around the stemlike part of the rearward part 301 being inserted in the ear tip 302. The stemlike part may slide in the elongatable ring 116, while the ring will be kept around the stemlike part by the ear tip 301 on the one hand, and another part of the rearward part 301 of the ear insert 300.

Adjacent the first extremity 111, the first member 110 has a stemlike part 117, ending at the opposite extremity 112 with a widened part 118, in which at the extremity 112, a recess 119 is provided. This first member is provided in its entirety from silicone, having a shore hardness of about 50. This recess 119 is the female half 115 or part of a male and female pair of connectors (114, 115).

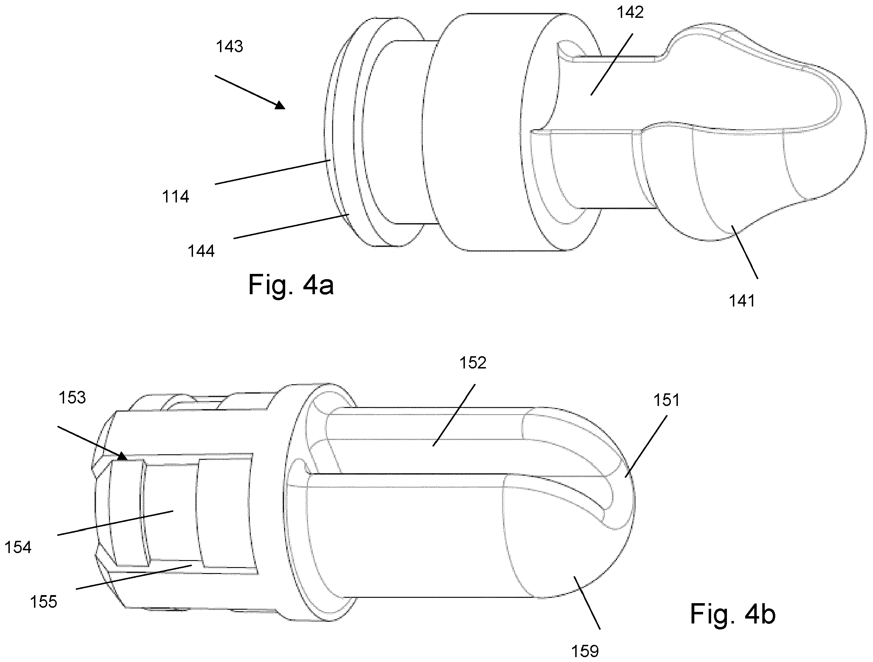

The interrupting means (100, 200) further comprises an aglet 113 for connecting the interrupting means to the end point (11, 12) of the flexible elongate object 10. The aglet 113 is provided, at its side 125 oriented away from the flexible elongate object 10, with a first half 114 of pair of mating connectors, in this embodiment the male half or part of a male/female pair of connectors. This male half 114, in detail shown in figure 4a, having an axial cross section being mushroom shaped. The cap 141 of the mushroom shape points away from the flexible elongate object 10. The female part of the connectors comprises a recess which is adapted to grip over the mushroom shaped point, and even may snap over this mushroom shaped point.

This mushroom shape comprises a radially inwards recess 142 in longitudinal direction of the mushroom shape along the outer surface of the mushroom shape. The cap 141 of the mushroom shape snugly fits into the recess 119, where it mates and connects, hence closes the pair of connectors. When a force is acting upon the male 114 or female half 115 in a direction parallel with the longitudinal direction of the interrupting means, hence the longitudinal direction of the flexible elongate object 10, the male and female part will disconnect as the male part will be pulled out of the female part, the mushroom shape leaving the recess. The radially inwards recess 142 will cause this disconnection to happen silently, the creation of a typical plopping noise being reduced or even avoided, when the male and female half disconnect. The mushroom shape is preferably provided from metal, most preferably aluminium. The friction coefficient between aluminium of this male part, and the silicone of the female part, was found to be beneficial to achieve the required interruption force needed, preferably being about 3N. The first half may have at its side 143 opposite to the mushroom shaped tip 141, a rib or ringlike protrusion 144, to provide a good grip when this side of the first half is pressed into the housing 127 of the aglet 113.

An alternative male half 154 is provided in figure 4b. This alternative male half 154 has front part 151 having the shape of a tapered cylinder. The top 159 of the tapered cylinder points away from the flexible elongate object 10. The female part of the connectors comprises a recess which is adapted to grip over this tapered cylinder. This male half also comprises a radially inwards recess 152 in longitudinal direction of the cylinder along the outer surface of the cylinder., and this to obtain the same effect as explained for the male half 114. The first half may have at its side 153 opposite to the top 159, a ribbed or profiled structure 155, to provide a good grip when this side of the first half is pressed into the housing 127 of the aglet 113.

This first half 114 is part of the aglet 113, which further comprises, at its opposite end, a housing 127 encompassing the end portion of the flexible elongate object 10. Adjacent the end point 11 respectively 12, a bush 121 is provided, preferably in cupper, which bush 121 encompasses the end part of the flexible elongate object 10. Pulling the flexible elongate object 10 outwards the hosing while the bush being in place, may cause the housing 127 to be clamped to the flexible elongate object, since the bush 121 will be squeezed and blocked between the flexible elongate object 10 and the inner side of the housing 127.

The first, mushroom shaped half 114 of the pair of mating connectors is press fitted in the opening of the housing 127 at the side 125 of the housing 127 oriented away from the flexible elongate object 10. Between the end of the flexible elongate object 11 in the housing 127 and the first, mushroom shaped half 114, a magnet 120 may be provided in the housing 127. The two magnets in the two aglets may attract one to the other when the two aglets are held adjacent and near one another.

It is clear that the ear insert 300 may as well be any other device to control or bring in a controlled way, sound or noise being provided to or into the ear channel, like an earbud, an ear headphone, true Wireless in-ear device (also referred to as TWS), Bluetooth ear insert, airpod and alike.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. An ear insert retainer (1) comprising a flexible elongate object (10) for linking two ear inserts, said flexible elongate object (10) having a first and a second end point (11, 12), one or both of said end points being adapted to be connected to a rearward part (301) of an ear insert (300), said ear insert retainer comprises at least a first interrupting means (100) along said flexible elongate object, said first interrupting means being adapted to interrupt the link of the ear insert with the flexible elongate object when a force applies to the flexible elongate object in the longitudinal direction.

2. An ear insert retainer according to claim 1, wherein the first interrupting means (100) being present at the first end point (11) of the flexible elongate object (10).

3. An ear insert retainer according to claim 2, wherein said first interrupting means (100) comprises a first member (110), said first member being adapted to movably and detachably connected the first end point (11) of the flexible elongate object (10) to a rearward part (301) of an ear insert

4. An ear insert retainer according to claim 3, wherein said first member (110) is a first elongate member having two extremities (111, 112), the first (111) of its two extremities being adapted to movably and detachably connected said first end point of the flexible elongate object to a rearward part of an ear insert.

5. An ear insert retainer according to claim 4, wherein said first member is provided from flexible polymeric material, preferably silicone and/or said first member is a stem-shaped member.

6. An ear insert retainer according to any one of the claims 4 or 5, said first interrupting means (100) further comprises an aglet (113) for connecting said first interrupting means to the flexible elongate object.

7. An ear insert retainer according to claim 6, wherein the aglet (113) is provided at its side oriented away from the flexible elongate object, with a first half (114) of pair of mating connectors.

8. An ear insert retainer according to claim 7, wherein said first half (114) of pair of mating connectors is the male half of a male and female pair of connectors, said male half having an axial cross section being mushroom shaped, the cap (141) of the mushroom shape pointing away from the flexible elongate object, said mushroom shape comprising a radially inwards recess (142) in longitudinal direction of the mushroom shape along the outer surface of the mushroom shape.

9. An ear insert retainer according to any one of the claims 7 or 8, wherein, the second half (115) of the mating connectors is provided at the second extremity (112) of said first member (110).

10. An ear insert retainer according to claim 9, wherein the second half (115) is provided from a polymer material, preferably flexible silicone polymer, the material of the male half having a higher hardness as compared to the material of the female half.

11. An ear insert retainer according to any one of the claims 2 to 10, wherein the ear insert retainer further comprises at least a second interrupting means (200) along said flexible elongate object, said second interrupting means being adapted to interrupt the link of the ear inserts when a force applies to the flexible elongate object in the longitudinal direction, said second interrupting means (200) being present at the second end point (12) of the flexible elongate object (10).

12. An ear insert retainer according to claim 11, wherein said first and second interrupting means are identical.

13. An ear insert retainer according to any one of the claims 11 or 12, wherein at least one of said first and second interrupting means comprises a magnetic element (120), the other of said first and second interrupting means comprises an element from magnetically attractable material, optionally being a magnet itself.

14. An ear insert set comprising at least two ear inserts and at least one ear insert retainer according to any one of the preceding claims.

15. The use of an ear insert retainer according to any one of the claims 1 to 13, to hold at least two ear inserts in linked to the other.
